# EUROPÄISCHE PATENTSCHRIFT

(11) **EP 0 743 366 B1**
(45) Veröffentlichungstag und Bekanntmachung des Hinweises auf die Patenterteilung: **11.07.2001**
(21) Anmeldenummer: 96107225.3
(22) Anmeldetag: 08.05.1996
(51) Int. Cl.: C12Q 1/04, C12N 7/00

(54) **Nachweis von Listerien mittels rekombinanter Bakteriophagen**
Determination of Listeria using recombinant bacteriophages
Méthode de détermination pour Listeria utilisant des bactériophages récombinants

(30) Priorität: 18.05.1995 DE 19517940
(43) Veröffentlichungstag der Anmeldung: 20.11.1996
(73) Patentinhaber: Xenogen Corporation, Alameda, CA 94501 (US)
(72) Erfinder: Lössner, Martin J., Dr., 85417 Marzling (DE); Scherer, Siegfried, Prof. Dr., 85354 Freising (DE)
(74) Vertreter: Jaenichen, Hans-Rainer, Dr.

(56) Entgegenhaltungen:
- WO-A-90/04041
- WO-A-93/16172
- JOURNAL OF GENERAL VIROLOGY, Bd. 75, 1994, Seiten 701-710, XP002008444 MARTIN J. LOESSNER ET AL., : "Structural proteins and DNA characteristics of 14 listeria typing bacteriophages"
- J. BACTERIOLOGY, Bd. 177, Nr. 22, 1995, Seiten 6601-6609, XP002008445 MARTIN J. LOESSNER, ET AL.,: "Organization and transcriptional analasys of the Listeria phage A115 late gene region comprising the major capsid and tail sheath protein genes cps and tsh."
- ROCOURT, J. ET AL.: "A multi-centre study on the phage typing of Listeria monocytogenes", ZBL. BAKT. HYG. , , 1985, Band , Nr. A259, Seiten 489 - 497
- GERNER-SMIDT, P. ET AL.: "A new Danish Listeria monocytogenes phage typing system", APMIS, , 1993, Band 101, Nr. , Seiten 160 - 167
- LOESSNER, M.: "Improved Procedure for Bacteriophage typing of Listeria strains and evaluation of new phages", APPLIED ENVIRONMENTAL MICROBIOLOGY, , Maerz 1991, Band 57, Nr. 3, Seiten 882 - 884
- LOESSNER. M ET AL.: "Taxonomical classification of 20 newly isolated Listeria Bacteriophages by electron microscopy and protein analysis", INTERVIROLOGY, , 1994, Band 37, Nr. , Seiten 31 - 35
- MCLAUCHLIN, J. ET AL.: "WHO study on subtyping Listeria monocytogenes: results of phage typing", INT. JOURNAL OF FOOD MICROBIOLOGY, , 1996, Band 32, Nr. , Seiten 289 - 299

## Beschreibung

Die Erfindung betrifft ein Nachweisverfahren für Bakterien der Gattung *Listeria,* sowie für dieses Nachweisverfahren geeignete Reagenzien.

Die Mitglieder der Gattung *Listeria* sind grampositive stäbchenförmige Bakterien, die ubiquitär vorkommen. Die Art *L. monocytogenes* ist pathogen für Menschen.

Die Ursache für Infektionen mit Listerien sind häufig kontaminierte Lebensmittel, in denen sich die Keime selbst bei niedrigen Temperaturen um 4 °C vermehren können. So wurden verschiedene Listerien-Epidemien auf den Verzehr von kontaminierten Nahrungsmitteln, wie z.B Rohmilch, Käse oder Krautsalat, zurückgeführt. Schnelle Nachweisverfahren für den Nachweis von Listerien, insbesondere in Lebensmitteln oder klinischen Proben, sind also dringend erforderlich.

Der Nachweis von Listerien erfolgt in bekannter Weise mittels Verfahren, die auf der Kultur der Mikroorganismen beruhen. Das in Int. J. Food Microbiol. **4** (1987), 249-256 beschriebene Verfahren dauert zwei Wochen. Ein etwas schnelleres Verfahren wird von der International Dairy Foundation (IDF) empfohlen; es dauert aber mindestens 6-8 Tage. Beide Verfahren sind wegen ihrer Dauer für eine schnelle Identifizierung ungeeignet. Beide Verfahren sind zudem arbeitsintensiv, da für die Gewinnung von Einzelkolonien Nährmedien mehrfach inokuliert werden müssen, und da anschließend die Isolate mittels biochemischer und serologischer Untersuchungsmethoden charakterisiert werden müssen.

Für die Anwendung von immunologischen Tests ist Voraussetzung, daß das Antigen exprimiert wird, was nicht für alle Proteine zu jeder Zeit zutrifft. Die Tests dauern zwar nur wenige Stunden, jedoch wird bei diesen Verfahren eine zweitägige Voranreicherungskultur benötigt, da die Erfassungsgrenze 10⁴ - 10⁶ Zellen beträgt. DNA-Sonden besitzen eine Erfassungsgrenze in derselben Größenordnung. Auch für diese Verfahren ist deswegen eine vorherige Vermehrung der Keime bis zur Erfassungsgrenze notwendig.

Die Polymerase-Ketten-Reaktion (Polymerase-Chain-Reaction: PCR) erlaubt die in-vitro Vermehrung von Nukleinsäuren. Die Erfassungsgrenze liegt bei 0,5 x 10³ Zellen. Eine eine Vorkultur ist bei diesem Verfahren im allgemeinen ausreichend, um beispielsweise gesetzlich vorgeschriebene Erfassungsgrenzen für den gesamte Nachweisreaktion sicherzustellen. Da jedoch auch DNA von toten Zellen oder auch isolierte DNA bei diesem Verfahren vermehrt wird, können Kontaminationen mit toten Zellen nicht von Kontaminationen mit lebenden Zellen unterschieden werden.

Aufgabe der vorliegenden Erfindung ist es, verbesserte Mittel und Methoden für den Nachweis von Bakterien der Gattung *Listeria* bereitzustellen.

Aus EP 0 168 933 (US 4,861,709) ist ein Nachweisverfahren für *Escherichia coli* offenbart, das auf der Verwendung eines rekombinanten Bakteriophagen beruht. Dieser Phage enthält das *lux*-Gen aus *Vibrio fischeri* und ermöglicht so den Nachweis von *E*. *coli* durch Biolumineszenz mit guter Erfassungsgrenze (0,5 x 10³ Zellen). Unter Verwendung von temperenten Phagen beschreiben G.J. Sarkis et al. (1995) Molecular Microbiology **15**, 1055 - 1067 ein Nachweisverfahren für Mykobakterien. Bei diesen Nachweisverfahren werden nur stoffwechselaktive Bakterienzellen erfaßt; Störungen durch tote Bakterienzellen wie bei der PCR-Technik treten nicht auf.

Gegenstand der Erfindung sind Verfahren zum Nachweis von Bakterien der Gattung *Listeria* in einer Probe umfassend die Schritte:
(a) Bereitstellen eines mittels Rekombinationstechniken hergestellten DNA-Vektors, enthaltend ein genetisches System, umfassend DNA, die die Expression eines oder mehrerer Proteine kodiert, wobei die Proteine kein Genprodukt von Bakterien der Gattung *Listeria* darstellen und das Vorhandensein der Proteine durch eine Nachweisreaktion festgestellt werden kann, und wobei der DNA-Vektor die Bakterien der Gattung *Listeria* infiziert und dabei das genetische System in die Bakterien über- tragen kann, und wobei als DNA-Vektor ein rekombinantor Listeria aus der Gruppe der Myoviren mit dem Morphotyp A1 verwendet wird;
(b) Vermischen der Probe mit besagtem DNA-Vektor unter Bedingungen, die eine Infektion von Bakterien der Gattung *Listeria* durch den DNA-Vektor erlauben;
(c) Exprimieren der nachweisbaren Proteine in den Bakterien der Gattung *Listeria*;
(d) Nachweis der nachweisbaren Proteine, wobei die Anwesenheit von Bakterien der Gattung *Listeria* nachgewiesen wird.

Zu DNA-Vektoren, wie sie in gängigen Lehrbüchern definiert werden (z.B: in Grundlagen und Praxis der Biotechnologie, H. Diekmann und H. Metz (1991); Gustav Fischer Verlag, Stuttgart, New York), gehören beispielsweise übertragbare DNA, Plasmide, Cosmide und Bakteriophagen. Als DNA-Vektor wird ein rekombinanter Bakteriophage aus der Gruppe der Myoviren mit dem Morphotyp A1 bevorzugt. In einer besonders bevorzugten Ausführungsform enthält dieser rekombinante Bakteriophage ein Gen, das für eine Luciferase aus Bakterien codiert, wobei die Expression der Luciferase durch Lichtemission nachgewiesen wird.

Gegenstand der Erfindung ist ein rekombinanter *Listeria*-Bakteriophage aus der Gruppe der Myoviren mit dem Morphotyp A1, dessen Genom ein Gen integriert enthält, das eine Luciferase aus Bakterien codiert, sowie dessen DNA.

Gegenstand der Erfindung sind auch Reagenzzusammenstellungen enthaltend neben einem Wachstumsmedium für Bakterien der Gattung *Listeria* infektiöse *Listeria*-Bakteriophagen aus der Gruppe der Myoviren mit dem Morphotyp A1, deren Genom ein Gen integriert enthält, das eine Luciferase aus Bakterien codiert.

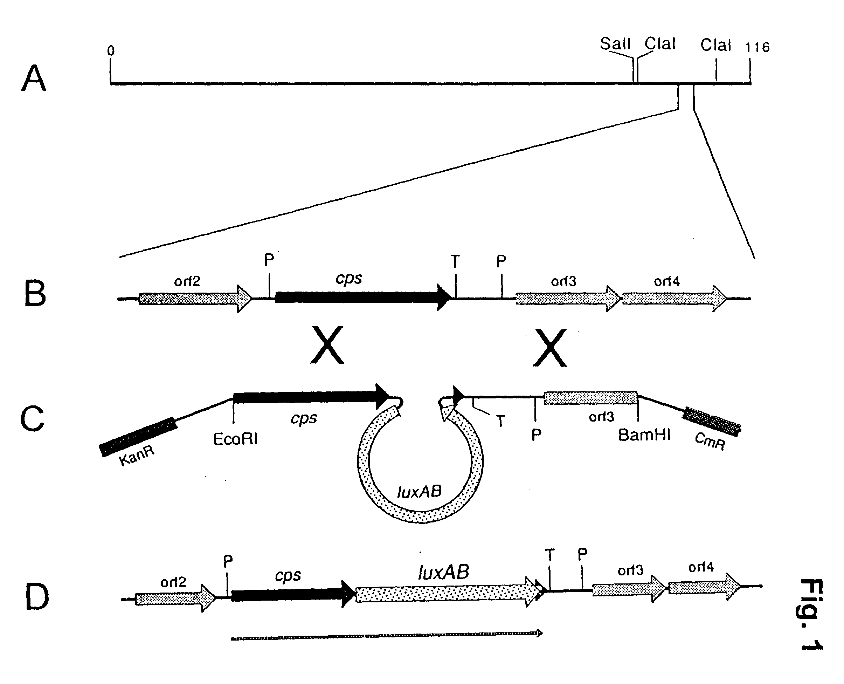

Abbildung 1 (A) zeigt einen Überblick über das Genom des *Listeria*-Bakteriophagen A511 (Wildtyp). Teil B zeigt einen Ausschnitt aus dieser Genkarte. Teil C die Insertion des Gens LUXAB in das Genom des Wildtyp-Phagen. Teil D zeigt das resultierende Transkript. Die Symbole P und T markieren Promotor- beziehungsweise Terminatorsequenzen; Schnittstellen von Restriktionsendonukleasen und Gene sind mit den üblichen Abkürzungen gekennzeichnet. Der zusätzliche Pfeil in Teil D kennzeichnet das Transkript *cps/lux*AB.

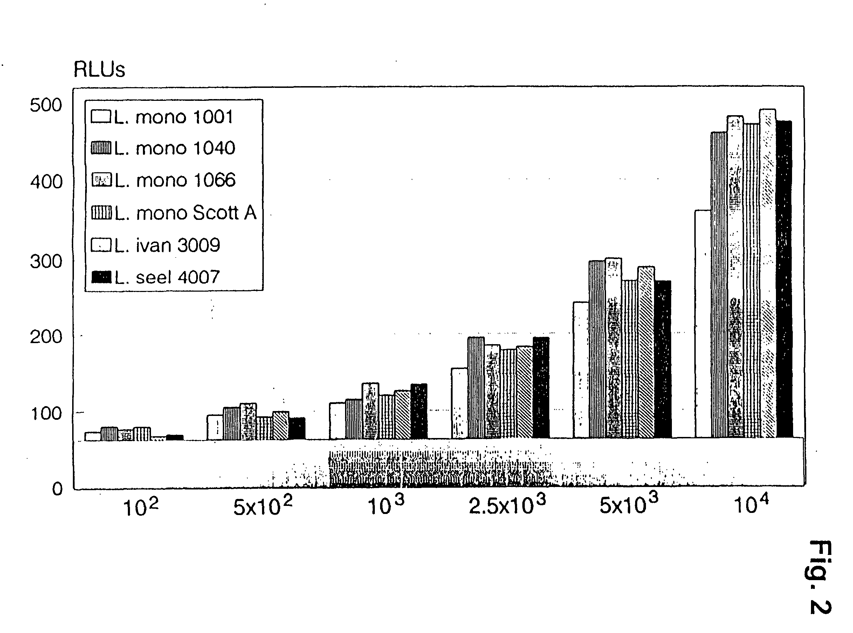

Abbildung 2 zeigt die Größe des Meßsignals (relative light unit: RLU) in Abhängigkeit von der Zellzahl für verschiedene Stämme von Listerien; darin bedeuten:
- L.mono:: *L. monocytogenes*
- L.ivan:: *L*. *ivanovii*
- L.seel:: *L. seeligeri*

Die graue Schraffung von 0 bis ca. 50 RLU kennzeichnet den unspezifischen Hintergrund des Meßsignals. An der Nachweisgrenze übersteigt das Meßsignal diesen unspezifischen Hintergrund.

Die Erfindung soll im folgenden näher beschrieben werden. Der *Listeria*-Bakteriophage A511, hinterlegt bei dem Referenzzentrum für bakterielle Viren (Reference Centre for Bacterial Viruses, Felix d'Herelle; Universität Laval, Québec, Kanada), lysiert spezifisch Bakterien der Gattung *Listeria* (obligat lytisch). Es handelt sich um einen komplex gebauten Myovirus (A1 Morphotyp). Bezüglich wesentlicher Merkmale unterscheidet sich der *Listeria*-Phage A511 von anderen bekannten *Listeria*-Phagen; die Unterschiede betreffen Morphologie, Wirtsbereich, Proteinprofile (Elektrophorese im SDS-Gel, isoelektrische Fokussierung, Aminosäure-Zusammensetzung der Hauptstrukturproteine, DNA/DNA Hybridisierung). Die Genomgröße von A511 beträgt etwa 116 kbp.

Für den Nachweis sind Markergene geeignet, die nicht in den Bakterien der Gattung *Listeria* vorkommen, und die mit Rekombinationstechniken in den Phagen A511 eingefügt werden. Solche Gene und ihre Genprodukte sind bekannt, dazu gehören das *lux*-Gen, das in Varianten in verschiedenen Leuchtbakterien, beispielsweise der Gattung *Vibrio*, vorkommt. Der Einbau des *lux*-Gens erlaubt den Nachweis durch Lumineszenzmessung. Bevorzugt wird das Gen *lux*AB aus Bakterien der Gattung *Vibrio,* insbesondere der Art *V. harveyi,* verwendet.

Erfindungsgemäß wurde das *lux*-Gen in einen stark exprimierten Genbereich des *Listeria*-Phagen A511 eingefügt. Dazu wurden die Haupt-proteine von Kapsid und Schwanzhülle identifiziert, indem die Proteine aus SDS-Gelen auf Trägermembranen transferiert (Western-Blot) wurden. Die Bestimmung der N-terminalen Aminosäuresequenzen ermöglichte die Herstellung spezifischer Oligonukleotide, mit deren Hilfe (Southern-Hybridisierung) entsprechende A511 DNA Fragmente mit den Genen für die gesuchten Proteine identifiziert werden konnten. Nach Subklonierung (in *E*. *coli* K-12) und Nukleotid-Sequenzierung von insgesamt 4 verschiedenen Fragmenten ergab sich eine kontinuierliche Sequenz von 10152 bp, welche neben neun weiteren offenen Leserahmen die Gene für das Haupt-Kapsid Protein (*cps*) und für das Schwanzhüllen Protein (*tsh*) enthält. Oberhalb von *cps* befindet sich ein starker, sogenannter "später" Promoter, welcher im Verlauf der Expression der Phagengene selektiv aktiviert wird, und zu sehr hoher Kopienzahl der entsprechenden mRNA Transkripte führt. 50 bp vor dem 3'-Ende von *cps* (Stop-Codon) liegt eine singuläre Schnittstelle für *Sna*Bl. An dieser Stelle kann somit ein "stumpfes" Schnittende erzeugt werden. Unterhalb des Gens (etwa 50 bp Abstand) befindet sich ein effektiver Transkriptions-Terminator.

Für die Konstruktion des erfindungsgemäßen rekombinanten Phagen A511::/*ux*AB wurde zunächst ein Fusionsprodukt der Luciferase-Gene (*lux*AB, ca. 2,1 kbp) von *Vibrio harveyi* mit geeigneten Translationssignalen (Ribosomen-Bindestelle, Zwischen-Region, Start-Codon) durch PCR subkloniert und direkt unterhalb von *cps* und vor dem Terminator in das Genom des Phagen A511 eingeführt. Folgende Einzelschritte wurden nach bekannten Methoden ausgeführt:
* Subklonieren (in *E*. *coli*) eines 2123 bp *Ssp*l Fragmentes (F3s) von A511 (die *Sna*Bl Schnittstelle liegt zentral) in die Smal Schnittstelle von pBluescriptll liefert pBS511-F3s.
* Amplifizieren von *lux*AB (vorhanden auf Promoter-Test Plasmid pSP331) mit spezifischen Primern. Der 5'-Primer (104 mer) enthält die 50 Nukleotide von *Sna*Bl Schnittstelle in *cps* bis Stop-Codon, optimierte Ribosomen-Bindestelle, Start-Codon und 35 Nukleotide vom 5'-Ende von *lux*AB. Der 3'-Primer (39 mer) enthält (komplementär) Stop-Codon und 25 Nukleotide vom 3'-Ende von *lux*AB. Primäre Amplifikation in der PCR mittels Taq-Polymerase; Zusatz von Pfu-Polymerase vor dem letzten Zyklus liefert erhöhten Anteil von Produkten mit stumpfen Enden.
* Einfügen des modifizierten *lux*AB Fragmentes in *Sna*BI geschnittenen pBS511-F3s liefert pBS511-F3s-*lux*AB. Überprüfen der korrekten Orientierung (Restriktions-Verdaus) und Integrität des 3'-Endes von *cps* (DNA Sequenzierung).
* Herausschneiden der F3s-flankierten *lux*AB Kassette mit *Bam*HI und *Eco*RI (vorhanden in MCS von pBluescript II).
* Einfügen der Kassette in den mit *Bam*HI/*Eco*RI geöffneten *E*.*coli-Listeria* Shuttle Vektor pCK 1 und Transformation in *E*. *coli* W3110 liefert pCK511-F3s-luxAB.
* Isolierung und Reinigung des Plasmides; Überprüfen korrekter Struktur und Orientierung.
* Elektrotransformation von pCK511-F3s-luxAB in *Listeria monocytogenes* WSLC 1001 und *Listeria ivanovii* WSLC 3009.
* Infektion von 1001(pCKS 11-F3s-luxAB) und 3009(pCKS511-F3s-luxAB) mit A511 (Wildtyp). Durch homologe Rekombination zwischen den *lux*AB-flankierenden A511 Fragmenten (F3s) und während der Infektion in der Zelle gebildeter A511 DNA kommt es zu Integration (doppeltes Crossover) von *lux*AB in das A511 Genom (Frequenz ca. 5 x 10⁻⁴) (siehe Abbildung 1).
* Anreicherung von rekombinanten Phagen in Lysaten von 3009(pCK511-F3sluxAB) mittels Verdünnungsmethode, bei der zunächst 20 Ansätze mit jeweils 5 x 10³ Phagen separat vermehrt werden. Positive Ansätze können anhand der *lux*AB Expression nach Infektion von 1001 oder 3009 Wirtszellen erkannt werden. Mehrfache Wiederholung dieses Verfahrens mit entsprechend gewählten Verdünnungen (bis hin zum einzelnen Plaque) ermöglicht Isolierung von A511::*lux*AB.
* Vermehrung von A511::luxAB auf Wirtstamm WSLC 3009; Waschen und Konzentrieren der Phagenpartikel durch Tangential-Flow Filtration (Entfernung des während der Morphogenese gebildeten Endolysins und der Luciferase AB); Konzentrationseinstellung auf ca. 10¹⁰ Plaqueformende Einheiten/ml.
* Isolierung und Restriktionsanalyse von A511::luxAB DNA, zur Bestätigung der korrekten Integration von luxAB (doppeltes Crossover statt singulärem Crossover mit Integration des gesamten Plasmids).

Aus den kompletten Phagen A511::luxAB kann nach dem Fachmann bekannten Methoden die DNA isoliert werden. Solche isolierte DNA kann ebenfalls als Vektor dienen.

Die experimentellen Bedingungen für die Expression von *lux*AB nach Phageninfektion wurden ermittelt. Die Expressionsparameter wurden unter Benutzung von Kulturen verschiedener Listeria Stämme (*L*. *monocytogenes* WSLC 1001 (Serovar 1/2c), ScottA (4b), *L. ivanoii* WSLC 3009) optimiert:
* Medium für Infektion: Hirn-Herz Bouillon (halbkonzentriert);
* *Listeria* Zellen in Log-Phase (Vor-Inkubation bei 30-35 °C) Verdünnungen anlegen (ca. 10⁷ Zellen/ml).
* 1 ml Zell-Suspension plus 30 µl Phagensuspension (= ca. 3 x 10⁸ Phagen/ml)
* Inkubationstemperatur: 18-20 °C (Luciferase ist Temperatur-sensitiv)
* Messung in Tube-Luminometer (LB9501, Fa. Berthold)
* Injektion von 50 µl 0,25 % Nonanal, Messung und Integration der RLUs für 5 s.
* Bester Messpunkt (maximale Lichtemission, gemessen in RLUs (Relative Light Units): ca. 120-130 min. post Infektion (bei 20 °C)

Kulturen verschiedener *Listeria* Stämme (*L*. *monocytogenes* WSLC 1001 (Serovar l/2c), WSLC 1040 (1/2a), WSLC 1066(1/2b), ScottA (4b), *L*. *ivanonii* WSLC 3009 (5), *L*. *seeligeri* WSLC 4007 (1/2b) wurden benutzt, um die Nachweisgrenze zu bestimmen; dabei wurde folgendermaßen vorgegangen:
* Verdünnungen anlegen (10², 5 x 10², 10³, 2,5 x 10³, 5 x 10³, 10⁴ Zellen/ml)
* Kontrollen:
   A) Ohne A511::luxAB (Null-Kontrolle)
   B) Ohne Zellen, ohne A511::luxAB (Hintergrund Kontrolle)
* Infektion nach optimalen Parametern (s.o.), Messung der RLUs (siehe Abbildung 2)
* Nachweisgrenze bei ca. 5 x 10² Zellen; zuverlässige Positiv-Grenze bei ca. 10³ Zellen.

In einem weiteren Versuch wurde der Nachweis von Listerien in einer Lebensmittelprobe untersucht; dazu wurde folgendermaßen vorgegangen:
* Künstliche Kontaminierung von Salatblättern (Eisbergsalat) mit 0 (= Kontrolle); 0,1; 1,0 und 10 Zellen *L. monocytogenes* ScottA/g Salat.
* Lagerung bei 4 °C für 1 und 7 Tage.
* Vermischung von je 25 g Salat mit 225 ml ANC-Bouillon oder *Listeria* Selektiv-Bouillon.
* Inkubation bei 30 °C für 24 Stunden.
* Kurze Sekundäranreicherung (nicht-selektiv): 1 ml Primäranreicherung plus 5 ml Hirn-Herz-Bouillon (halbkonzentriert); 3-4 Std. Inkubation bei 30-35 °C.
* Infektion von 1 ml Test-Kultur nach optimalen Parametern (s.o.); Messung der RLUs.
* Ergebnis: Der Ansatz mit 0,1 Zellen *L. monocytogenes* ScottA pro Gramm Salat ergibt eindeutig positives Signal nach 1 und 7 Tagen Lagerung des Salates.

## Patentansprüche

1. Verfahren zum Nachweis von Bakterien der Gattung *Listeria* in einer Probe umfassend die Schritte:
(a) Bereitstellen eines mittels Rekombinationstechniken hergestellten DNA-Vektors, enthaltend ein genetisches System, umfassend DNA, die die Expression eines oder mehrerer Proteine kodiert, wobei die Proteine kein Genprodukt von Bakterien der Gattung *Listeria* darstellen und das Vorhandensein der Proteine durch eine Nachweisreaktion festgestellt werden kann, und wobei der DNA-Vektor die Bakterien der Gattung *Listeria* infiziert und dabei das genetische System in die Bakterien übertragen kann, und wobei als DNA-Vektor ein rekombinanter *Listeria*-Bakteriophage aus der Gruppe der Myoviren mit dem Morphotyp A1 verwendet wird;
(b) Vermischen der Probe mit besagtem DNA-Vektor unter Bedingungen, die eine Infektion von Bakterien der Gattung *Listeria* durch den DNA-Vektor erlauben;
(c) Exprimieren der nachweisbaren Proteine in den Bakterien der Gattung *Listeria;*
(d) Nachweis der nachweisbaren Proteine, wobei die Anwesenheit von Bakterien der Gattung *Listeria* nachgewiesen wird.

2. Verfahren nach Anspruch 1, dadurch gekennzeichnet, daß das genetische System eine Luciferase als nachweisbares Protein codiert, wobei die Luciferase in den Bakterien durch Lichtemission nachgewiesen wird.

3. Rekombinanter *Listeria*-Bakteriophage aus der Gruppe der Myoviren mit dem Morphotyp A1, dadurch gekennzeichnet, daß in dem Genom des Bakteriophagen ein Gen integriert ist, das eine Luciferase codiert.

4. Isolierte DNA erhältlich durch Extraktion aus einem Bakteriophagen mit den Merkmalen des Anspruches 3.

5. Reagenzzusammenstellung enthaltend neben einem Wachstumsmedium für Bakterien der Gattung *Listeria* infektiöse *Listeria*-Bakteriophagen mit den Merkmalen des Anspruches 4.

## Claims

1. A method for detecting bacteria of the genus *Listeria* in a sample comprising the following steps:
(a) providing a DNA vector produced by means of recombination techniques, containing a genetic system, comprising DNA which encodes the expression of one or more proteins, wherein the proteins are no gene product of bacteria of the genus *Listeria*, and wherein the presence of the proteins can be determined by a detection reaction and wherein the DNA vector infects the bacteria of the genus *Listeria*, thus, being able to transmit the genetic system into the bacteria, and wherein a recombinant *Listeria* bacteriophage from the group of the myoviruses having the morpho type A1 is used as DNA vector;
(b) mixing of the sample with said DNA vector under conditions allowing for an infection of bacteria of the genus *Listeria* by means of the DNA vector;
(c) expressing the detectable proteins in bacteria of the genus *Listeria;*
(d) detection of the detectable proteins, wherein the presence of bacteria of the genus *Listeria* is detected.

2. The method according to claim 1, characterised in that the genetic system encodes a luciferase as detectable protein, wherein the luciferase is detected in the bacteria by means of light emission.

3. Recombinant *Listeria* bacteriophage from the group of the myoviruses having the morpho type A1 characterised in that a gene encoding a luciferase is integrated in the genome of the bacteriophage.

4. Isolated DNA obtainable by extraction from a bacteriophage with the features of claim 3.

5. Reagent combination containing, in addition to a growth medium for bacteria of the genus *Listeria*, infectious *Listeria* bacteriophages having the features of claim 4.

## Revendications

1. Procédé pour la mise en évidence de bactéries de l'espèce *listeria* dans une éprouvette, comprenant les étapes consistant à :
(a) mettre à disposition un vecteur d'ADN préparé au moyen des techniques recombinantes, contenant un système génétique, comprenant l'ADN qui code l'expression d'une ou de plusieurs protéines, les protéines ne représentant aucun produit génique des bactéries de l'espèce *listeria* et la présence des protéines pouvant être constatée par une réaction de mise en évidence et le vecteur d'ADN infectant les bactéries de l'espèce *listeria* et permettant ainsi de transférer le système génétique dans les bactéries et en utilisant comme vecteur d'ADN un bactériophage de *listeria* recombiné du groupe des myovirus ayant le morphotype A1;
(b) mélanger l'éprouvette avec ledit vecteur d'ADN dans des conditions qui permettent une infection des bactéries de l'espèce *listeria* par le vecteur d'ADN;
(c) exprimer les protéines mises en évidence dans les bactéries de l'espèce *listeria;*
(d) mettre en évidence les protéines décelables, la présence des bactéries de l'espèce *listeria* étant mise en évidence.

2. Procédé selon la revendication 1, caractérisé en ce que le système génétique code une luciférase en tant que protéine décelable, la luciférase dans les bactéries étant mise en évidence par émission lumineuse.

3. Bactériophage de *listeria* recombiné du groupe des myovirus ayant le morphotype A1, caractérisé en ce que dans le génome du bactériophage est intégré un gène qui code une luciférase.

4. ADN isolé pouvant être obtenu par extraction à partir d'un bactériophage ayant les caractéristiques de la revendication 3.

5. Combinaison d'agents réactifs contenant en plus d'un milieu de croissance pour bactéries de l'espèce *listeria*, des bactériophages de *listeria* infectieux ayant les caractéristiques de la revendication 4.
